# EUROPÄISCHE PATENTANMELDUNG

(11) **EP 2 110 161 A1**
(43) Veröffentlichungstag der Anmeldung: **21.10.2009**
(21) Anmeldenummer: 09154947.7
(22) Anmeldetag: 12.03.2009
(51) Int. Cl.: A61N 5/10

(54) **Vorrichtung zur Durchführung einer Bestrahlung und Verfahren zur Überwachung einer solchen**

(30) Priorität: 16.04.2008 DE 102008019128; 16.04.2008 US 45310
(71) Anmelder: Siemens Aktiengesellschaft, 80333 München (DE)
(72) Erfinder: Rietzel, Eike, 64289, Darmstadt (DE)

(57) **Zusammenfassung**

Vorrichtung zur Durchführung einer Bestrahlung und Verfahren zur Überwachung einer solchenDie Erfindung betrifft eine Vorrichtung und ein Verfahren zur Durchführung bzw. zur Überwachung einer Bestrahlung eines sich bewegenden Objektes mit einem Partikelstrahl, wobei
- von einem Strahlaustritt ein Partikelstrahl auf das sich bewegende Objekt gerichtet wird,
- von einer Bildgebungseinheit, umfassend einen Röntgendetektor und einen gegenüberliegenden Röntgenstrahler, die unabhängig von der Position des Strahlaustritts um das Objekt positionierbar sind, während der Applikation des Partikelstrahls Röntgenbilder aus unterschiedlichen Richtungen aufgezeichnet werden und hieraus Online eine Serie digitaler Tomosynthese-Bilder von dem sich bewegenden Objekt rekonstruiert werden, und
- die rekonstruierten digitalen Tomosynthese-Bilder ausgewertet werden, so dass Bewegung des sich bewegenden Objekts erfasst wird und der Verlauf der Bestrahlung gesteuert wird.

## Beschreibung

Die Erfindung betrifft eine Vorrichtung zur Durchführung einer Bestrahlung eines sich bewegenden Objektes sowie ein Verfahren zur Überwachung einer derartigen Bestrahlungsbehandlung.

In der Strahlentherapie wird unter anderem erkranktes Gewebe mit Röntgenstrahlen, mit Elektronenstrahlen oder mit Partikelstrahlen bestrahlt. Insbesondere die Partikeltherapie entwickelt sich in den letzten Jahren zu einem etablierten Verfahren zur Behandlung von Gewebe, insbesondere von Tumorerkrankungen. Bestrahlungsverfahren, wie sie im Rahmen der Partikeltherapie eingesetzt werden, können jedoch auch in nichttherapeutischen Bereichen eingesetzt werden, wie beispielsweise bei der Bestrahlung von Phantomen oder nicht-lebenden Körpern im Rahmen von Forschungsarbeiten, bei der Bestrahlung von Materialien, etc.

Bei der Partikeltherapie werden Partikel erzeugt, vornehmlich Ionen wie Protonen, Kohlenstoffionen, oder andere Ionensorten. Diese Partikel werden auf hohe Energien in einem Beschleuniger beschleunigt, zu einem Partikelstrahl geformt und anschließend auf das zu bestrahlende Gewebe gerichtet. Die Partikel dringen in das zu bestrahlende Gewebe ein und geben dort in einem umschriebenen Bereich ihre Energie ab. Die Eindringtiefe des Partikelstrahls in das zu bestrahlende Gewebe hängt vornehmlich von der Energie des Partikelstrahls ab. Je höher die Energie des Partikelstrahls ist, desto tiefer dringen die Partikel in das zu bestrahlende Gewebe ein.

Während einer Bestrahlung muss eine Bewegung des zu bestrahlenden Objekts berücksichtigt werden. Beispielsweise können Atembewegungen zu einer wesentlichen Positionsveränderung von Lungen- und Lebertumoren führen, so dass das Zielvolumen gegebenenfalls nicht wie gewünscht bestrahlt wird. Bei intensitätsmodulierten Bestrahlungen kann es zusätzlich zu Interferenzeffekten zwischen Bestrahlung und Bewegung des Zielvolumens kommen, so dass eine geplante Dosisbelegung des Zielvolumens beeinträchtigt wird.

Zur Steuerung des Bestrahlungsverlaufs bei sich bewegenden Zielvolumina sind unterschiedliche Verfahren bekannt. Beispielsweise versteht man unter dem so genannten "gating", dass die Bewegung des Zielvolumens direkt oder indirekt erfasst wird. Eine Bestrahlung des Zielvolumens findet nur dann statt, wenn sich das Zielvolumen an einem gewünschten Ort befindet. Unter dem so genannten "tracking" versteht man die Nachführung des Partikelstrahls.

Die Bewegung des Zielvolumens kann mit verschiedenen Verfahren erfasst werden, beispielsweise über externe Marker am Patienten, durch eine Beobachtung der Patientenoberfläche, oder mithilfe von Gürtel oder Spyrometer, die die Atembewegung erfassen. Eine andere Möglichkeit ist es, kontinuierlich Röntgenbilder beziehungsweise Durchleuchtungsbilder von dem zu bestrahlenden Objekt aufzunehmen. Hierdurch kann die Bewegung des zu bestrahlenden Objekts auch intern erfasst werden. Dadurch lassen sich mögliche Fehler aufgrund von Unterschieden zwischen externer und interner Bewegung vermeiden.

Aus der US 7,245,698 B2 ist ein System zu Bestrahlung mit Röntgenstrahlen bekannt, bei dem digitale Tomosynthese-Aufnahmen von dem zu bestrahlenden Objekt rekonstruiert werden.

Es ist die Aufgabe der Erfindung, eine Vorrichtung zur Durchführung einer Bestrahlungsbehandlung mit einem Partikelstrahl anzugeben, mit der eine mögliche Bewegung des zu bestrahlenden Objekts flexibel und genau erfasst werden kann. Weiterhin ist es die Aufgabe der Erfindung, ein ebensolches Verfahren zur Überwachung einer Bestrahlungsbehandlung anzugeben.

Die Aufgabe der Erfindung wird gelöst durch eine Vorrichtung nach Anspruch 1 sowie durch ein Verfahren nach Anspruch 10. Vorteilhafte Weiterbildungen der Erfindung finden sich in den Merkmalen der abhängigen Ansprüche.

Die erfindungsgemäße Vorrichtung zur Durchführung einer Bestrahlung eines sich bewegenden Objektes mit einem Behandlungsstrahl, weist auf:
- einen Strahlaustritt, von dem aus ein Behandlungsstrahl, insbesondere ein auf Bestrahlungsenergie beschleunigter Partikelstrahl, auf das sich bewegende Objekt richtbar ist,
- eine Bildgebungseinheit, umfassend einen Röntgendetektor und einen gegenüberliegenden Röntgenstrahler, die unabhängig von der Position des Strahlaustritts um das Objekt positionierbar sind,
wobei die Bildgebungseinheit dazu ausgebildet ist, während einer Applikation des Behandlungsstrahls Röntgenbilder aus unterschiedlichen Richtungen aufzuzeichnen und hieraus online eine Serie digitaler Tomosynthese-Bilder von dem sich bewegenden Objekt zu rekonstruieren, und
- eine Steuerungseinheit, die dazu ausgebildet ist, die digitalen Tomosynthese-Bilder der Serie auszuwerten und hierauf basierend den Verlauf der Bestrahlung zu steuern.

Digitale Tomosynthese-Bilder sind lediglich zweidimensionale Bilder, die sich jedoch - anders als Röntgenbilder, d.h. Durchleuchtungsbilder - durch einen guten Kontrast für Weichteilgewebe auszeichnen. Unter lediglich zweidimensionalen Bildern sind auch Bilder zu verstehen, bei denen Rekonstruktion zwar zunächst ein dreidimensionaler Bild-Datensatz erzeugt wird, der jedoch lediglich in einer Ebene ein scharfes Bild enthält, im Gegensatz zu echten dreidimensionalen Bilddatensätzen wie sie z.B. aus der Cone-Beam-Computertomographie bekannt sind. Der gewünschte Weichteilkontrast ist bei einem derartigen dreidimensionalen Datensatz bei dem Bild in dieser Ebene wiedergegeben. Die anderen Ebenen im dreidimensionalen Datensatz weisen dann eine geringe Bildqualität auf. Durch die Auswertung der digitalen Tomosynthese-Bilder kann eine Bewegung des sich bewegenden Objektes erfasst werden. Im Vergleich zu herkömmlichen Röntgenbildern können beispielsweise auch Bewegungen von Weichteilen genau erfasst werden. Die erfasste Bewegung eines Zielvolumens kann dann dazu verwendet werden, den Verlauf der Bestrahlung zu steuern. Bei herkömmlichen Röntgenbildern ist im Gegensatz dazu oftmals nur die Erfassung von Hochkontrast-Objekten wie beispielsweise Knochen möglich.

Im Gegensatz zur Erzeugung dreidimensionaler Bilddatensätze, wie beispielsweise durch die so genannte Cone-Beam- Computertomographie, ist die Aufnahme von Röntgenbildern zur Rekonstruktion von digitalen Tomosynthese-Bildern und die Rekonstruktion der Tomosynthese-Bilder um ein Vielfaches schneller, vor allem weil Daten nur über einen kleineren Winkelbereich aufgenommen werden. Die zeitliche Auflösung, die mit zweidimensionalen digitalen Tomosynthese-Bildern zur Verfolgung der Bewegung eines zu bestrahlenden Objekts erreicht werden kann, ist folglich ebenso deutlich höher. Die zweidimensionalen digitalen Tomosynthese-Bilder eignen sich daher besonders zur Erfassung der tatsächlichen, das heißt der aktuelle Bewegung, des Objekts.

Beispielsweise kann nun auch bei einer Bestrahlung von Leber-oder Lungentumoren der Tumor bzw. dessen Bewegung direkt erfasst werden und hierauf basierend der Bestrahlungsverlaufs gesteuert werden. Diese Information führt zu einer genaueren Erfassung der Tumorbewegung und somit zu einer verbesserten Bestrahlungsgenauigkeit für den Fall, dass die Bestrahlung der Bewegung angepasst ist. Die Vorteile können speziell bei Lungen- und Lebertumoren von entscheidender Bedeutung sein, da die Tumoren direkt während eine Bestrahlung visualisiert werden können.

Der Verlauf der Bestrahlung kann derart kontrolliert werden, dass eine Bestrahlung unterbrochen wird (gating), falls sich das zu bestrahlende Objekt zu weit von einer vordefinierten Sollposition weg bewegt. Alternativ und/oder zusätzlich kann die Richtung des Partikelstrahls beziehungsweise die Eindringtiefe des Partikelstrahls durch Ablenkung des Partikelstrahls beziehungsweise durch Anpassung der Energie des Partikelstrahls entsprechend nachgeregelt werden, so dass der Partikelstrahl der Bewegung des zu bestrahlenden Objekts nachgeführt wird (tracking).

Der Röntgendetektor und der Röntgenstrahler können während der kontinuierlichen Bewegung beispielsweise hin und her geschwenkt werden oder durchgreifende, zum Beispiel komplexere und in sich wiederkehrende Bewegungen ausführen.

Während des gesamten Vorgangs und insbesondere während der Applikation des Partikelstrahls kann die Position des Strahlaustritts räumlich fest bleiben, da die Bildgebungseinheit unabhängig vom Strahlaustritt positionierbar ist. Die Positionierung der Bildgebungseinheit ist somit von der Position des Strahlaustritts entkoppelt. Dies ermöglicht eine flexible Ausgestaltung der Vorrichtung. So kann die Vorrichtung in einem Bestrahlungsraum zum Einsatz kommen, der einen raumfesten Strahlaustritt aufweist. Andererseits kann die Vorrichtung auch in einem Bestrahlungsraum zum Einsatz kommen, bei dem der Strahlaustritt über eine Gantry in verschiedene Positionen gebracht werden kann. Üblicherweise bleibt in einem derartigen Bestrahlungsraum der Strahlaustritt während der Applikation des Partikelstrahls ebenfalls raumfest.

Bei der Online-Rekonstruktion der digitalen Tomosynthese-Bilder entsteht eine zeitliche Serie der digitalen Tomosynthese-Bilder, in der die aktuelle Bewegung des sich bewegenden Objekts abgebildet ist. Bei der Rekonstruktion eines der Tomosynthese-Bilder können beispielsweise n im Wesentlichen aufeinander folgende Röntgenbilder, die aus unterschiedlichen Richtungen aufgezeichnet wurden, verwendet werden. n kennzeichnet dabei die Anzahl von Röntgenbildern, die benötigt wird, um eine Tomosynthese in der gewünschten Qualität zu ermöglichen. Je höher die Anzahl n ist, desto mehr Rohdaten stehen für das Tomosynthese-Bild zur Verfügung, so dass die Qualität des Tomosynthese-Bildes gesteigert werden kann. Je höher die Anzahl n ist, desto geringer wird jedoch die zeitliche Auflösung, die durch das Tomosynthese-Bild dargestellt wird, vor allem weil die zugehörigen Röntgenbilder über einen längeren Zeitraum aufgenommen wurden. Im Allgemeinen wird daher ein Kompromiss gewählt, der der gewünschten Qualität der Tomosynthese und der gewünschten zeitlichen Auflösung gerecht wird.

Die Rekonstruktion der Tomosynthese-Bilder kann dabei kontinuierlich erfolgen, wobei jeweils die benötigte Anzahl n an Röntgenbildern, d.h. an Projektionen, zu Grunde liegt. Die verschiedenen Tomosynthese-Bilder müssen hinsichtlich ihrer Rohdaten (Röntgenbilder) nicht unabhängig sein. Beispielsweise können zur Rekonstruktion eines Tomosynthese-Bildes n aufeinander folgende Röntgenbilder als Rohdaten verwendet werden. Bei der Rekonstruktion des darauf folgenden Tomosynthese-Bildes können eines oder mehrere zeitlich nachfolgende Röntgenbilder zu den n aufeinander folgenden Röntgenbildern hinzugefügt werden, während die gleiche Anzahl der Röntgenbilder zu Beginn der aufeinander folgenden Röntgenbilder entfernt wird. Wenn sich der Röntgendetektor und der Röntgenstrahler beispielsweise kreisförmig um den Partikelstrahl drehen, kann immer die benötigte Anzahl n an Bildern aus einem Ring-Buffer entnommen werden.

In einer bevorzugten Ausführungsvariante ist die Bildgebungseinheit dazu ausgebildet, den Röntgendetektor beziehungsweise den gegenüberliegenden Röntgenstrahler in einer Präzessionsbewegung spiralartig oder kreisartig zu bewegen, das heißt die Röntgenstrahlachse führt eine kreis- oder spiralförmige Präzessionsbewegung aus. Hierdurch können Röntgenbilder aus vielen unterschiedlichen Raumrichtungen aufgenommen werden. Da eine kreis- oder spiralförmige Präzessionsbewegung im Allgemeinen regelmäßig ist, lässt sich auf einfache Weise eine kontinuierliche Online-Rekonstruktion der Tomosynthese-Bilder implementieren, da die Regelmäßigkeit der Bewegung ausgenutzt werden kann.

In einer bevorzugten Ausführungsvariante wird eine Anzahl der Röntgenbilder, die zur Rekonstruktion eines der Tomosynthese-Bilder verwendet werden, der Bewegung des Objektes angepasst. Unter der Bewegung des Objektes kann dabei eine tatsächliche Bewegung oder aber auch eine zu erwartende Bewegung - beispielsweise aufgrund von Erfahrungswerten oder von Voruntersuchungen - verstanden werden. Alternativ und/oder zusätzlich ist es möglich, die Geschwindigkeit, mit der der Röntgendetektor und der Röntgenstrahler um das zu bestrahlende Objekt bewegt werden, der Bewegung des Objektes anzupassen. So kann z.B. bei einem sich schneller bewegenden Objekt die Geschwindigkeit des Röntgendetektors und des Röntgenstrahlers erhöht werden, um trotz der schnelleren Objektbewegung noch eine ausreichende zeitliche Auflösung zu erhalten.

In einer anderen Ausführungsvariante ist die Rekonstruktionsebene, entlang derer zumindest eines der Tomosynthese-Bilder rekonstruiert wird, frei wählbar. Bei der Aufzeichnung der Bilddaten wird die Positionierung des Röntgendetektors und des Röntgenstrahlers entsprechend angepasst. Dadurch kann die Rekonstruktionsebene so gelegt werden, dass eine Bewegung des Zielobjekts optimal erfasst werden kann. Beispielsweise kann die Rekonstruktionsebene so gewählt werden, dass die räumliche Richtung der Bewegung des Objekts in der Rekonstruktionsebene liegt. Auf diese Weise ist eine Objektbewegung besonders gut durch eine Auswertung der Tomosynthese-Bilder in der Rekonstruktionsebene ermittelbar.

Bevorzugterweise sind der Röntgendetektor und der gegenüberliegende Röntgenstrahler an einem Tragarm angeordnet, der über ein robotisches Positioniersystem im Raum positionierbar ist. Hierdurch ist eine besonders flexible Positionierung der Bildgebungseinheit möglich. Zudem erlaubt das robotische Positioniersystem üblicherweise eine sehr schnelle Bewegung des Röntgendetektors und des gegenüberliegenden Röntgenstrahlers, so dass auch schnelle Bewegungen des zu bestrahlenden Objekts sicher und genau erfasst werden können.

Das erfindungsgemäße Verfahren zur Überwachung einer Bestrahlungsbehandlung eines sich bewegenden Objektes mit einem Behandlungsstrahl, insbesondere mit einem Partikelstrahl, weist folgende Schritte auf:
- Aufzeichnen von Röntgenbildern aus unterschiedlichen Richtungen von dem sich bewegenden Objekt während der Applikation eines Behandlungsstrahls aus einer festen Raumrichtung,
- direkte Online-Rekonstruktion einer Serie digitaler Tomosynthese-Bilder aus den aufgezeichneten Röntgenbildern,
- Erfassen der Bewegung des sich bewegenden Objektes durch Auswertung der digitalen Tomosynthese-Bilder,
- Steuern des Bestrahlungsverlaufs abhängig von der erfassten Bewegung.

Das Verfahren kann wie die Vorrichtung entsprechend weitergebildet werden.

Ausführungsformen der Erfindung mit vorteilhaften Weiterbildungen gemäß den Merkmalen der abhängigen Ansprüche werden anhand der folgenden Zeichnung näher erläutert, ohne jedoch darauf beschränkt zu sein. Es zeigen:
Fig. 1 einen schematischen Aufbau einer Partikeltherapieanlage,
Fig. 2 eine Anordnung eines Strahlaustritts und eine Bildgebungseinheit in einem Bestrahlungsraum,
Fig. 3 ein Schema zur Illustration der Online-Rekonstruktion einer zeitlichen Serie von digitalen Tomosynthese-Bildern aus einer zeitlichen Serie von Röntgenbildern, und
Fig. 4 eine schematische Darstellung von Verfahrensschritten, die bei einer Ausführungsform des erfindungsgemäßen Verfahrens durchgeführt werden.

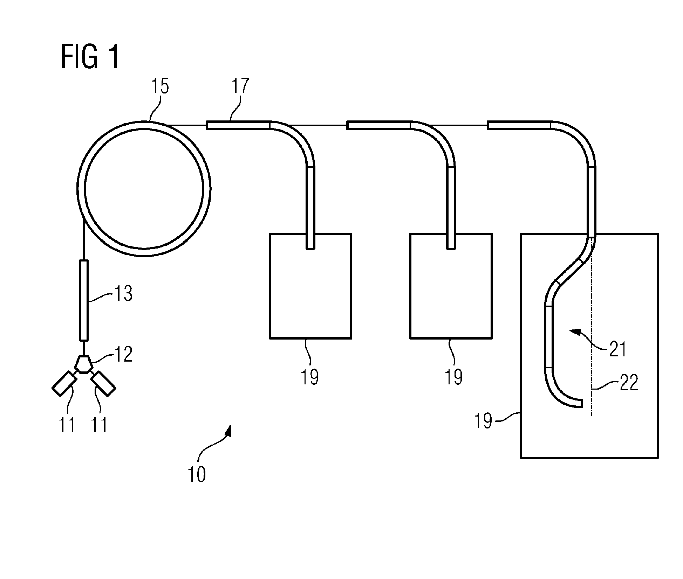

Fig. 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Aufbau einer Partikeltherapieanlage 10 ist typisch für viele Partikeltherapieanlagen, kann aber auch hiervon abweichen. Die nachfolgend beschriebenen Ausführungsbeispiele sind sowohl in Zusammenhang mit der anhand von Fig. 1 dargestellten Partikeltherapieanlage als auch mit anderen Partikeltherapieanlagen einsetzbar.

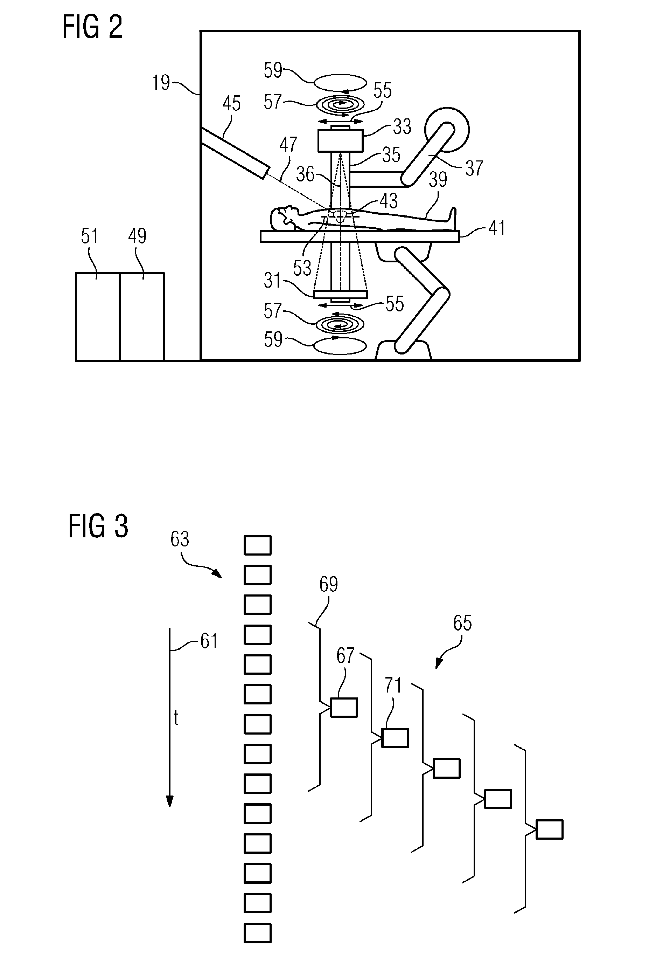

Fig. 2 zeigt eine mögliche Anordnung eines Strahlaustritts und der Bildgebungseinheit in einem Bestrahlungsraum.

Die Bildgebungseinheit umfasst einen Röntgendetektor 31 und einen Röntgenstrahler 33, die an einem Tragarm 35, z.B. einem C-Bogen, einander gegenüber liegend angeordnet sind. Der Tragarm 35 selbst ist mithilfe eines Roboterarms 37, beispielsweise mithilfe eines sechsachsigen Knickarm-Roboters, im Raum flexibel positionierbar. Mithilfe des Röntgendetektors 31 und des Röntgenstrahlers 33 können Röntgenaufnahmen, zum Beispiel Durchleuchtungsaufnahmen, von einem Patienten 39 aufgenommen werden, der zur Bestrahlung auf einer Patientenliege 41 positioniert ist. Insbesondere das zu bestrahlende Zielvolumen 43, beispielsweise ein zu bestrahlendes Organ, kann in den Durchleuchtungsaufnahmen abgebildet sein.

Alternativ zu der Ausführungsvariante mit dem Tragarm können auch der Röntgendetektor und der Röntgenstrahler unabhängig voneinander positioniert werden, beispielsweise über zwei Roboterarme. Dies kann eine erhöhte Flexibilität ermöglichen, da kein starrer Tragarm zwischen dem Röntgenstrahler und dem Röntgendetektor angeordnet ist.

Es ist sogar denkbar, lediglich entweder den Röntgendetektor oder den Röntgenstrahlen beweglich zu positionieren und den anderen sogar statisch. Beispielsweise kann sich der Röntgendetektor bewegen und eine bewegliche Blende bei dem Röntgenstrahler dafür sorgen, dass sich die Röntgenstrahlen unterschiedlich eingeblendet werden.

Zur Bestrahlung tritt aus einem Strahlaustritt 45 ein Partikelstrahl 47 aus und ist auf den Patienten 39 gerichtet. Hier gezeigt ist ein im Raum räumlich fest installierter Strahlaustritt 45. Alternativ ist es auch möglich, den Strahlaustritt 45 an einer drehbaren Gantry zu befestigen, so dass der Strahlaustritt 45 um den Patienten 39 gedreht werden kann. Während der Applikation des Partikelstrahls 47 bleibt der Strahlaustritt 45 jedoch im Allgemeinen ortsfest.

Die Positionierung des Röntgendetektors 31 und des Röntgenstrahlers 33 kann unabhängig von dem Strahlaustritt 45 durchgeführt werden. Während der Applikation des Partikelstrahls 47 wird der Tragarm 35 durch den Roboterarm 37 hin und her bewegt, wobei eine Serie von Durchleuchtungsbildern aufgezeichnet wird. Aus der Serie von Durchleuchtungsbildern wird "online", das heißt während der Applikation des Partikelstrahls 47, eine Serie von digitalen Tomosynthese-Bildern rekonstruiert (auch als "on the fly"-Rekonstruktion bezeichnet). Hierzu werden die aufgezeichneten Durchleuchtungsbilder einer Rechnereinheit 49 weitergeleitet, in der die Rekonstruktion der Tomosynthese-Bilder stattfindet.

In der Serie von digitalen Tomosynthese-Bildern kann nun die Bewegung des Zielvolumens 43, das heißt während der Applikation des Partikelstrahls 47, ausgewertet werden. Diese Auswertung findet ebenfalls "on the fly" statt. Diese so gewonnene Information wird dazu verwendet, den Verlauf der Bestrahlung zu steuern. Die Auswertung und die Steuerung des Verlaufs der Bestrahlung finden in einer Steuerungseinheit 51 statt. Beispielsweise kann der Partikelstrahl 47 abgeschaltet werden, sobald das zu bestrahlende Zielvolumen 43 sich nicht mehr in einer gewünschten Position befindet und gegebenenfalls wieder eingeschaltet werden, sobald das zu bestrahlende Zielvolumen 43 sich wieder in der gewünschten Position befindet. Alternativ und/oder zusätzlich ist es möglich, den Partikelstrahl 47 einer Bewegung des Zielvolumens 43 nachzuführen, falls die Bewegung des Zielvolumens 43 innerhalb bestimmter Grenzen stattfindet.

Die Steuerungseinheit 51 und/oder die Rechnereinheit 49 zur Bildrekonstruktion können in einer einzigen Rechnereinheit implementiert sein oder auch auf verschiedene Untereinheiten aufgesplittet, als eigenständige Einheiten implementiert werden oder in einer Steuerungseinheit für die gesamte Partikeltherapieanlage.

Wenn der Tragarm 35 wie hier dargestellt in etwa senkrecht positioniert ist, befindet sich die Rekonstruktionsebene 53 der Tomosynthese-Bilder in etwa senkrecht hierzu, das heißt in einer horizontalen Richtung. Je nach abzubildendem Zielvolumen beziehungsweise dessen Bewegung kann jedoch auch eine andere Lage der Rekonstruktionsebene 53 gewählt werden. Die Rekonstruktionsebene 53 sollte so gewählt werden, dass die Bewegung des Organs möglichst gut erfasst und aufgelöst werden kann.

Der Tragarm 35 kann auf verschiedene Art und Weise bewegt werden. Eine einfache Bewegung ist durch einen Doppelpfeil 55 angedeutet und entspricht einer Schwenkbewegung. Eine andere Möglichkeit, den Tragarm 35 zu bewegen, ist eine kreis- oder spiralförmige Bewegung, angedeutet durch die Spirale 57 oder durch den Kreis 59. Bei letzten beiden Bewegungen führt die Röntgenstrahlachse 36 eine Präzessionsbewegung aus.

Fig. 3 veranschaulicht, wie aus der zeitlichen Serie von Durchleuchtungsbildern 63 eine zeitliche Serie von digitalen Tomosynthese-Bildern 65 rekonstruiert wird. Der Zeitverlauf ist durch den Pfeil 61 angedeutet.

Um z.B. ein erstes Tomosynthese-Bild 67 zu rekonstruieren, wird eine erste Gruppe 69 von n aufeinander folgenden Durchleuchtungsbildern verwendet. In dem ersten Tomosynthese-Bild 67 ist daher ein Querschnitt durch das abzubildende Organ zu einem ersten Zeitpunkt abgebildet. Die eigentliche Rekonstruktion des Tomosynthese-Bildes 67 aus der ersten Gruppe 69 von ein aufeinander folgenden Durchleuchtungsbildern, die aus unterschiedlichen Richtungen aufgezeichnet worden, kann mit bekannten Verfahren erfolgen.

Um das nächste, zeitlich folgende Tomosynthese-Bild 71 zu rekonstruieren, wird zu der ersten Gruppe 69 von n aufeinander folgenden Durchleuchtungsbildern ein (oder mehrere) folgende Durchleuchtungsbilder hinzugefügt, während eine gleiche Anzahl von zeitlich älteren Durchleuchtungsbildern entfernt werden. Das nächste Tomosynthese-Bild 71 bildet daher einen Querschnitt durch das Organ zu einem späteren Zeitpunkt ab. Auf diese Weise fortfahrend lässt sich aus der zeitlichen Serie von Durchleuchtungsbildern 63 eine zeitliche Serie von Tomosynthese-Bildern 65 erzeugen. Insbesondere bedeutet dies, dass die Tomosynthese-Bilder 65 hinsichtlich ihrer Rohdaten, das heißt ihrer Durchleuchtungsbilder 63 nicht unabhängig voneinander sind bzw. sein müssen.

Die zeitliche Auflösung der Tomosynthese-Bilder 65 beziehungsweise die Genauigkeit der Abbildung in den Tomosynthese-Bildern 65 hängt unter anderem von der Geschwindigkeit der Aufzeichnung der Durchleuchtungsbilder 63, von der Geschwindigkeit der Richtungsänderung bei der Aufzeichnung der Durchleuchtungsbilder 63 und von der Anzahl n der verwendeten Durchleuchtungsbilder bei der Rekonstruktion ab. Diese Parameter können daher jeweils auf die Bedürfnisse eingestellt werden.

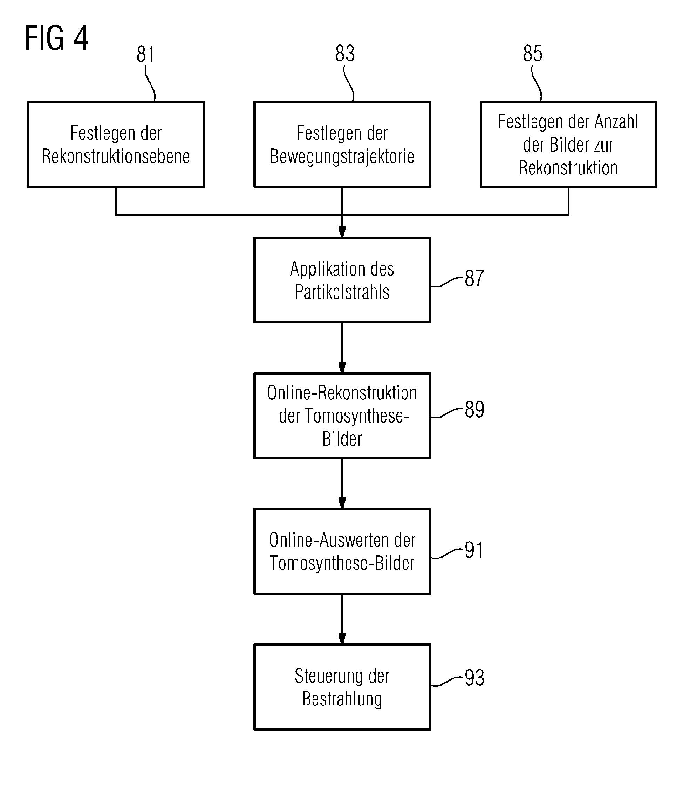

Fig. 4 zeigt eine schematische Übersicht über Verfahrensschritte.

Zunächst wird eine räumliche Lage der Rekonstruktionsebene festgelegt (Schritt 81). Die Rekonstruktionsebene kann dabei derart gewählt werden, dass eine Organbewegung möglichst gut in der Rekonstruktionsebene erfasst wird. Dies ist ohne weiteres möglich, da der Strahlaustritt und die Positionierung der Bildgebungseinheit räumlich voneinander entkoppelt sind.

Weiterhin wird eine Bewegungstrajektorie des Röntgendetektors und des Röntgenstrahlers festgelegt (Schritt 83), zum Beispiel der räumliche Verlauf der Bewegungstrajektorie und/oder die Geschwindigkeit der Bewegung. Insbesondere die Geschwindigkeit der Bewegung der Bildgebungsvorrichtung kann der Bewegung des Organs angepasst sein. Dies kann beispielsweise die tatsächliche Bewegung des Organs - gemessen mit geeigneten Mitteln - sein, oder die zu erwartende Bewegung, die typischerweise bei dem Organ auftritt. Hierdurch lässt sich eine gewünschte zeitliche Auflösung bei der nachfolgenden Rekonstruktion der Tomosynthese-Bilder erreichen.

Weiterhin wird zudem die Anzahl der Durchleuchtungsbilder festgelegt (Schritt 85), die zur Rekonstruktion eines der Tomosynthese-Bilder verwendet werden. Die Anzahl der Durchleuchtungsbilder ist der Bewegungstrajektorie und/oder der Bewegungsgeschwindigkeit des Röntgendetektors und des Röntgenstrahlers, der gewünschten Genauigkeit der Rekonstruktion und/oder insbesondere der Bewegung des abzubildenden Organs angepasst.

Das Festlegen der Rekonstruktionsebene, der Bewegungstrajektorie und/oder der Anzahl der Durchleuchtungsbilder zur Rekonstruktion kann beispielsweise interaktiv durch einen Anwender erfolgen. Es ist jedoch auch möglich, hierzu auf Parametereinstellungen zurückzugreifen, die zuvor abgespeichert worden sind. Beispielsweise kann bei der Bestrahlung eines bestimmten Organs wie der Lunge auf eine bestimmte Parametereinstellung zurückgegriffen werden, bei der Bestrahlung eines anderen Organs wie der Prostata auf eine andere Parametereinstellung, und so weiter. Auch patientenspezifische Einstellungen, die bei wiederkehrenden Bestrahlungen verwendet werden, sind bevorzugt zu verwenden. In den Parametereinstellungen sind jeweils Werte hinterlegt, die den Bedürfnissen angepasst sind.

Während einer anschließenden Bestrahlung erfolgt eine Applikation des Partikelstrahls (Schritt 87). Während diese Applikation geschieht, wird mit dem Röntgenstrahler und mit dem Röntgendetektor eine zeitliche Serie von Durchleuchtungsbildern aus unterschiedlichen Richtungen aufgezeichnet. Online (oder "on the fly"), das heißt während der Applikation des Partikelstrahls, erfolgt eine Rekonstruktion einer zeitlichen Serie von digitalen Tomosynthese-Bildern (Schritt 89). Eine Auswertung der zeitlichen Serie der digitalen Tomosynthese-Bilder erlaubt es, die Bewegung des zu bestrahlenden Organs zu erfassen (Schritt 91). Die erfasste Bewegung wird dazu verwendet, den Bestrahlungsverlauf zu steuern (Schritt 93).

## Patentansprüche

1. Vorrichtung zur Durchführung einer Bestrahlung eines sich bewegenden Objektes (43), aufweisend:
- einen Strahlaustritt (45), von dem aus ein Behandlungsstrahl (47) auf das sich bewegende Objekt (43) richtbar ist,
- eine Bildgebungseinheit, umfassend einen Röntgendetektor (31) und einen gegenüber liegenden Röntgenstrahler (33), die unabhängig von der Position des Strahlaustritts (45) um das Objekt (43) positionierbar sind,
wobei die Bildgebungseinheit dazu ausgebildet ist, während einer Applikation des Behandlungsstrahls (47) Röntgenbilder (63) aus unterschiedlichen Richtungen aufzuzeichnen und hieraus online eine Serie digitaler Tomosynthese-Bilder (65) von dem sich bewegenden Objekt (43) zu rekonstruieren, und
- eine Steuerungseinheit (51), die dazu ausgebildet ist, zumindest einen Teil der digitalen Tomosynthese-Bilder (65) auszuwerten und hierauf basierend einen Verlauf der Bestrahlung zu steuern.

2. Vorrichtung nach Anspruch 1, wobei
die Bildgebungseinheit dazu ausgebildet ist, eine Bewegung durchzuführen, bei der eine Röntgenstrahlachse (36) eine kreis- oder spiralartige Präzessionsbewegung ausführt.

3. Vorrichtung nach Anspruch 1 oder 2, wobei
eine Anzahl der Röntgenbilder (63), die zur Rekonstruktion eines der Tomosynthese-Bilder (65) verwendet werden, einer tatsächlichen Bewegung oder einer zu erwartenden Bewegung des Objektes (43) angepasst ist.

4. Vorrichtung nach einem der Ansprüche 1 bis 3, wobei
eine Geschwindigkeit, mit der der Röntgendetektor (31) und der Röntgenstrahler (33) um das Objekt (63) bewegt werden, einer tatsächlichen Bewegung oder einer zu erwartenden Bewegung des Objektes (43) angepasst ist.

5. Vorrichtung nach einem der Ansprüche 1 bis 4, wobei eine Rekonstruktionsebene (53), entlang derer zumindest eines der Tomosynthese-Bilder (65) rekonstruiert wird, frei wählbar ist.

6. Vorrichtung nach Anspruch 5, wobei die Rekonstruktionsebene (53) einer räumlichen Richtung einer tatsächlichen Bewegung oder einer zu erwartenden Bewegung des Objektes (43) angepasst ist.

7. Vorrichtung nach einem der Ansprüche 1 bis 6, wobei der Röntgendetektor (31) und der gegenüberliegende Röntgenstrahler (33) an einem Tragarm (35) angeordnet sind.

8. Vorrichtung nach einem der Ansprüche 1 bis 7, wobei zur Positionierung des Röntgendetektors (31) und/oder des Röntgenstrahlers (33) im Raum zumindest ein Roboterarm (37) verwendet wird.

9. Vorrichtung nach einem der Ansprüche 1 bis 8, wobei der Behandlungsstrahl ein Partikelstrahl ist und der Strahlaustritt während der Applikation des Partikelstrahls räumlich ortsfest bleibt.

10. Verfahren zur Überwachung einer Bestrahlungsbehandlung eines sich bewegenden Objektes mit einem Behandlungsstrahl (47), aufweisend folgende Schritte:
- Aufzeichnen von Röntgenbildern (63) aus unterschiedlichen Richtungen von dem sich bewegenden Objekt (43) während der Applikation eines Behandlungsstrahls (47) aus einer festen Raumrichtung,
- direkte Online-Rekonstruktion einer Serie digitaler Tomosynthese-Bilder (65) aus den aufgezeichneten Röntgenbildern (63),
- Erfassen der Bewegung des Objektes (43) durch Auswertung der digitalen Tomosynthese-Bilder (65),
- Steuern des Bestrahlungsverlaufs abhängig von der erfassten Bewegung.

11. Verfahren nach Anspruch 10, wobei
beim Aufzeichnen der Röntgenbilder (63) aus unterschiedlichen Richtungen ein Röntgendetektor (31) und ein gegenüber liegender Röntgenstrahler (33) in einer kreisartigen oder spiralartigen Präzessionsbewegung um das Objekt (73) bewegt wird.

12. Verfahren nach Anspruch 10 oder 11, wobei
bei der direkten Online-Rekonstruktion eine Anzahl der Röntgenbilder (63), die zur Rekonstruktion eines der Tomosynthese-Bilder (65) verwendet werden, einer tatsächlichen Bewegung oder einer zu erwartenden Bewegung des Objektes (43) angepasst wird.

13. Verfahren nach einem der Ansprüche 10 bis 12, wobei
beim Aufzeichnen der Röntgenbilder (63) aus unterschiedlichen Richtungen eine Geschwindigkeit, mit der ein Röntgendetektor (31) und ein gegenüberliegender Röntgenstrahler (35) um das sich bewegende Objekt (43) bewegt werden, einer tatsächlichen Bewegung oder einer zu erwartenden Bewegung des Objektes (43) angepasst wird.

14. Verfahren nach einem der Ansprüche 10 bis 13, wobei eine Rekonstruktionsebene (53), entlang derer zumindest eines der Tomosynthese-Bilder (65) rekonstruiert wird, frei wählbar ist.

15. Verfahren nach Anspruch 14, wobei die Rekonstruktionsebene (53) abhängig von einer räumlichen Richtung einer tatsächlichen Bewegung oder einer zu erwartenden Bewegung des Objektes (43) gewählt wird.

16. Verfahren nach einem der Ansprüche 10 bis 15, wobei
der Behandlungsstrahl ein Partikelstrahl ist.
